# FASCICULE DE BREVET EUROPEEN

(11) **EP 2 996 590 B1**
(45) Date de publication et mention de la délivrance du brevet: **22.03.2017**
(21) Numéro de dépôt: 14721980.2
(22) Date de dépôt: 10.04.2014
(51) Int. Cl.: A61B 17/70

(54) **ENSEMBLE DE REPRISE POUR UN MATERIEL D'OSTEOSYNTHESE VERTEBRALE**
PRÜFANORDNUNG FÜR EIN ELEMENT EINER WIRBEL-OSTEOSYNTHESE-VORRICHTUNG
REVISION ASSEMBLY FOR AN ITEM OF VERTEBRAL OSTEOSYNTHESIS EQUIPMENT

(30) Priorité: 19.04.2013 FR 1353592
(43) Date de publication de la demande: 23.03.2016
(73) Titulaire: Medicrea International, 69140 Rillieux-la-Pape (FR)
(72) Inventeur: SOURNAC, Denys, F-01600 Reyrieux (FR); MOSNIER, Thomas, F-69270 Rochetaillée Sur Saône (FR); RYAN, David, F-69660 Collonges au Mont d'Or (FR)
(74) Mandataire: Jeannet, Olivier
(86) Numéro de dépôt international: PCT/IB2014/060614
(87) Numéro de publication internationale: WO 2014/170803

(56) Documents cités:
- WO-A1-98/55038
- FR-A1- 2 856 581
- US-B2- 8 308 775

## Description

La présente invention concerne un ensemble de reprise pour un matériel d'ostéosynthèse vertébrale.

Un matériel d'ostéosynthèse vertébrale comprend, de manière bien connue en soi :
- au moins une barre rigide destinée à relier plusieurs vertèbres,
- des organes d'ancrage osseux, tels que des vis ou des crochets lamaires,
- des pièces de connexion permettant de relier la ou les barres aux organes d'ancrage osseux, et
- des moyens de serrage permettant d'opérer une traction sur les organes d'ancrage osseux de manière à réaliser une correction de la position des vertèbres par rapport à la référence que constitue la barre. Le matériel, une fois mis en place, permet d'immobiliser les vertèbres dans leur position de correction.

Des matériels connus, de ce type, sont décrits notamment par les publications de demande de brevet ou de brevet N° WO 98/55038 et N° US 8,308,775, ou encore par la publication de demande de brevet français N° FR 2 856 581 A1. Dans ces matériels, chaque organe d'ancrage osseux est sous la forme d'une vis pédiculaire "polyaxiale", c'est-à-dire comprenant un pion fileté proximal articulé ; la pièce de connexion correspondante est destinée à être engagée sur ce pion fileté proximal au moyen d'un trou qu'elle comprend ; ledit moyen de serrage est constitué par un écrou destiné à être vissé sur le pion proximal fileté et à prendre appui contre la pièce de connexion. Le serrage de l'écrou permet de tirer la vis en direction de la pièce de connexion jusqu'à venue d'une surface d'appui aménagée sur la vis au contact de la pièce de connexion. Cette traction progressive permet de réaliser la correction de la position de vertèbres. Une fois ladite surface d'appui placée contre la pièce de connexion, l'écrou est serré à bloc et la partie du pion dépassant au-delà de l'écrou est cassée. Pour favoriser cette cassure, le pion comprend une portion de section réduite, de moindre résistance ; après cassure, le pion proximal fileté se réduit à une portion subsistante de ce pion.

Avec de tels matériels incluant des vis pédiculaires, il existe le problème de parfaitement positionner ces vis dans les pédicules des vertèbres. En effet, une vis, si elle est déviée par rapport à sa position adéquate, peut conduire à une compression voire à une lésion d'une terminaison nerveuse, générant une douleur importante pour le patient. Dans un tel cas, il est nécessaire de réopérer le patient afin de corriger la position de la vis. Cette correction peut cependant conduire à un changement notable de la position de la vis par rapport à la pièce de connexion correspondante, rendant difficile, voire impossible, la remise en place de l'écrou sur la portion subsistante du pion fileté.

Le même problème de remise en place de l'écrou existe lorsque la vis comprend un pion proximal court non cassable, c'est-à-dire un matériel dont la capacité de réduction repose essentiellement sur l'ancillaire associé à ce matériel.

Les documents précités, et en particulier le document FR 2 856 581 A1, n'abordent pas ce problème spécifique et ne fournissent donc pas de solution à celui-ci. En l'absence d'une telle solution, soit il peut être décidé de ne pas rectifier la position incorrecte d'un organe d'ancrage osseux, soit il est nécessaire de remplacer l'ensemble du matériel, ce qui implique une procédure longue et complexe.

La présente invention vise à remédier à ce problème spécifique.

L'ensemble de reprise qu'elle concerne est utilisable avec un matériel d'ostéosynthèse vertébrale comprenant au moins un organe d'ancrage osseux incluant un pion proximal fileté, au moins une pièce de connexion comprenant un trou pour son engagement sur ce pion proximal fileté, et au moins un écrou destiné à être vissé sur le filet que comprend extérieurement le pion pour la réception de cet écrou.

Selon l'invention,
- l'ensemble de reprise comprend une tige filetée de prolongation présentant une extrémité élargie qui délimite une cavité axiale taraudée, cette cavité axiale taraudée étant dimensionnée pour permettre le vissage de la tige filetée de prolongation sur ledit filet que comprend le pion fileté ;
- le trou que comprend ladite pièce de connexion a un diamètre tel qu'il peut recevoir ladite extrémité élargie de la tige filetée de prolongation au travers de lui ; et
- l'ensemble de reprise comprend un écrou dit "de remontage", apte à être vissé sur cette tige filetée de prolongation.

Ainsi, la tige filetée de prolongation est apte à être engagée au travers du trou que comprend la pièce de connexion puis à être vissée sur la portion subsistante du pion fileté, et ledit écrou de remontage est mis en place sur la tige filetée de prolongation. L'ensemble de reprise selon l'invention permet donc de réaliser à nouveau un serrage de la pièce de connexion contre l'organe d'ancrage osseux, au moyen de cet écrou de remontage.

Le trou que comprend ladite pièce de connexion, ou que comprend chaque pièce de connexion lorsque le matériel comprend plusieurs pièces de connexion, a un diamètre supérieur à celui d'une pièce de connexion classique, étant donné qu'il est destiné à recevoir non seulement le pion fileté au travers de lui mais également ladite extrémité élargie de la tige filetée de prolongation.

L'écrou de remontage peut être l'écrou d'origine lui-même si la tige filetée de prolongation a le même diamètre et présente un filet identique à celui du pion proximal fileté ; cet écrou est, sinon, un écrou de remplacement.

L'ensemble de reprise s'utilise comme suit :
- desserrage de l'écrou d'origine de l'organe d'ancrage à repositionner et dégagement de la pièce de connexion associée à cet organe ;
- repositionnement de l'organe d'ancrage ;
- remise en place de la pièce de connexion sur le pion fileté ;
- vissage de la tige filetée de prolongation sur ce pion fileté, par engagement de ladite extrémité élargie au travers du trou que comprend la pièce de connexion ;
- mise en place de l'écrou de remontage, et
- section de la tige filetée de prolongation au-delà de cet écrou.

L'organe d'ancrage peut être une vis pédiculaire ou un crochet lamaire ; une telle vis pédiculaire peut être "monoaxiale", c'est-à-dire dans laquelle le pion proximal fileté fait corps avec le reste de la vis ou "polyaxiale", c'est-à-dire dans laquelle le pion proximal fileté est articulé par rapport au reste de la vis ; dans un tel cas, le pion proximal fileté comprend des moyens pour son immobilisation en rotation lors de la mise en place de la tige filetée de prolongation. Ces moyens peuvent être constitués par une tête d'articulation à section polygonale placée dans une cavité de forme correspondante, par exemple telle que décrite par la publication de demande de brevet N° FR 2 831 048 ; ces moyens peuvent également être constitués par une collerette solidaire du pion, comprenant un ou plusieurs méplats, et par une forme correspondante du trou de la pièce de connexion, tel que cela est décrit dans la publication de demande de brevet internationale N° WO 98/55038.

De préférence, la tige filetée de prolongation comprend une portion de section réduite favorisant sa cassure au-delà de la face proximale de l'écrou de remontage, après complet serrage de ce dernier.

La portion proximale de la tige filetée de prolongation est ainsi apte à être aisément cassée au-delà de cette face proximale une fois la mise en place réalisée.

Lorsque, comme cela est fréquemment le cas, le trou de la pièce de connexion forme une cuvette proximale destinée à recevoir portion distale conique de l'écrou d'origine, ainsi que cela est décrit dans la publication de demande de brevet internationale N° WO 98/55038 précitée, l'écrou d'origine ne pourra pas être réutilisé car ladite extrémité élargie de la tige filetée de prolongation occupe une portion substantielle de l'espace délimité par ladite cuvette une fois mise en place sur le pion proximal fileté. Ledit écrou de remplacement comprend alors une base distale élargie permettant une prise d'appui contre la face proximale de la pièce de connexion.

Cet écrou de remplacement peut cependant comprendre une portion distale tubulaire, conique extérieurement, faisant saillie de ladite base distale élargie et dimensionnée pour prendre place dans l'espace subsistant entre la pièce de connexion et ladite extrémité élargie de la tige fileté de prolongation.

Cette portion distale tubulaire permet ainsi d'occuper ledit espace subsistant et d'assurer une parfaite reprise du jeu entre la pièce de connexion et ladite tige filetée de prolongation.

L'invention sera bien comprise, et d'autres caractéristiques et avantages de celle ci apparaîtront, en référence au dessin schématique annexé, représentant, à titre d'exemple non limitatif, une forme de réalisation préférée de l'ensemble de reprise concerné.
La figure 1 est une vue en perspective d'une tige filetée de prolongation et d'un écrou de remplacement constituant cet ensemble de reprise ;
la figure 2 est une vue de cette tige et de cet écrou de remplacement en coupe passant par l'axe de cette tige et de cet écrou ;
les figures 3 et 4 sont des vues, respectivement en perspective et en coupe partielle, de différentes pièces faisant partie d'un matériel d'ostéosynthèse vertébrale, au cours d'une première étape d'une méthode de reprise de la position d'une vis d'ancrage pédiculaire "polyaxiale" que comprend ce matériel ; ladite coupe partielle passe par l'axe d'un trou que comprend une pièce de connexion faisant partie de ce matériel ;
les figures 5, 7 et 9 sont des vues du matériel similaires à la figure 3, au cours d'étapes successives subséquentes de la méthode de reprise ;
les figures 6, 8 et 10 sont des vues de ce matériel similaires à la figure 4, au cours de ces mêmes étapes, et
la figure 11 est une vue du matériel similaire à la figure 6 dans le cas d'une vis d'ancrage pédiculaire "monoaxiale".

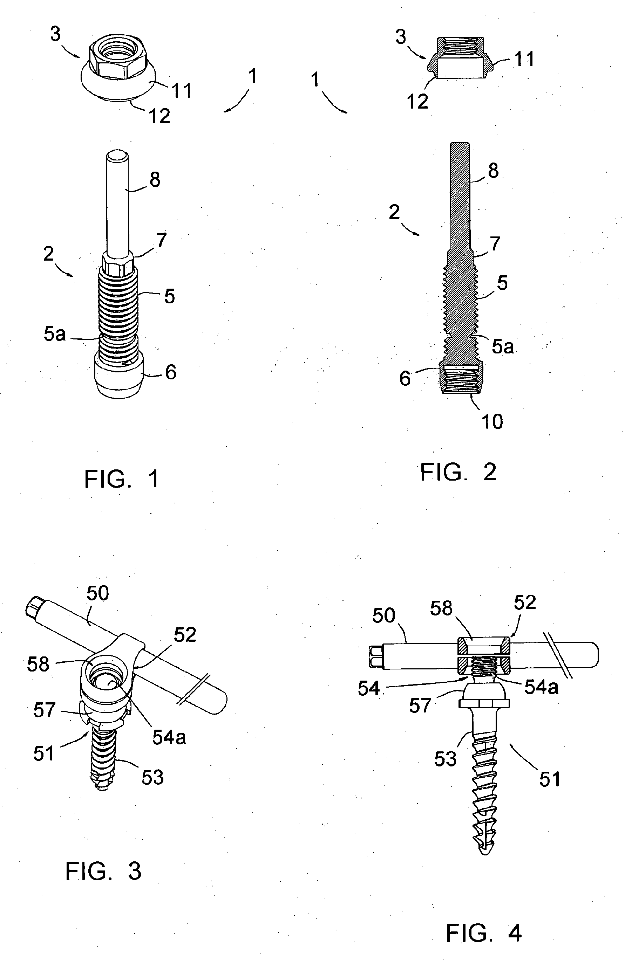

Les figures 1 et 2 représentent une tige filetée de prolongation 2 et un écrou dit "de remplacement" 3 constituant conjointement un ensemble 1 permettant la reprise de la position d'une vis d'ancrage pédiculaire que comprend un matériel d'ostéosynthèse vertébrale.

Ce matériel d'ostéosynthèse vertébrale est de l'un des types bien connus décrits notamment par les publications de demande de brevet ou de brevet N° WO 98/55038 et N° US 8,308,775. Comme visible sur les figures 3 et 4, ce matériel comprend au moins une barre rigide 50 destinée à relier plusieurs vertèbres les unes aux autres, plusieurs vis pédiculaires 51 dites "polyaxiales", c'est-à-dire incluant des pions proximaux filetés articulés 54, plusieurs pièces de connexion 52 comprenant des trous 58 pour leur engagement sur les pions 54, et des écrous, dits "d'origine", destinés à être vissés sur ces pions proximaux filetés 54 pour tirer les vis 51 progressivement en direction des pièces de connexion 52 jusqu'à venue de surfaces d'appui 57 que comprennent ces vis 51 contre les pièces de connexion 52, réalisant ainsi une correction de la position de vertèbres. Une fois lesdites surfaces d'appui 57 placées contre les pièces de connexion 52, lesdits écrous d'origine sont serrés à bloc et les parties des pions 54 dépassant au-delà de ces écrous sont cassées. Pour favoriser cette cassure, chaque pion comprend une portion de section réduite, de moindre résistance, de sorte qu'après mise en place du matériel, le pion proximal fileté 54 se réduit à une portion 54a subsistante de ce pion.

Les figures 3 et 4 montrent une partie seulement d'un matériel d'ostéosynthèse vertébrale de ce type, à savoir : l'une des barres rigides 50, l'une des vis pédiculaires 51 et une pièce de connexion 52.

La vis pédiculaire 51 comprend une embase 53 de vissage dans le pédicule d'une vertèbre et le pion proximal fileté articulé 54 ; sur les figures 3 et 4, c'est la portion subsistante 54a de ce pion proximal fileté articulé 54 qui est représentée.

L'embase 53 forme une cavité proximale de réception d'une tête d'articulation distale que forme le pion 54. La tête d'articulation est du type de celle décrite par la publication de demande de brevet N° FR 2 831 048, à section polygonale placée dans une cavité de forme correspondante. Pour constituer la vis 51, la tête d'articulation est engagée dans la cavité puis la paroi 57 de l'embase 53 délimitant la périphérie de la cavité est sertie autour de cette tête d'articulation, selon une forme extérieure sensiblement hémisphérique, ainsi que cela est visible sur la figure 4. Après sertissage, l'ensemble est tel que la tête d'articulation soit retenue dans ladite cavité et que le pion 54 ait un débattement multidirectionnel par rapport à l'embase 53 tout en étant calé en rotation selon son axe par rapport à cette embase.

Le pion 54 présente une forme cylindrique et est fileté au niveau de sa.face périphérique. Il forme la portion de section réduite précitée, dont la cassure crée ladite portion subsistante 54a.

La paroi 57 forme quant à elle une surface d'appui de l'embase 53 contre la pièce de connexion 52 lorsque l'écrou précité est complètement serré.

La pièce de connexion 52 forme un trou 58 permettant son engagement sur le pion 54. Ce trou 58 a cependant un diamètre plus large que celui nécessaire au passage du pion 54: il permet de plus le passage d'une extrémité élargie 6 que comprend la tige de prolongation 6, comme cela est décrit ci-dessous.

En référence à nouveau aux figures 1 et 2, il apparaît que la tige 2 comprend une portion filetée 5, une extrémité élargie 6 située à une extrémité de cette portion filetée 5, une portion à facettes 7 à l'autre extrémité de la portion 5 et une portion lisse 8 de plus faible section, reliée à la portion 7 du côté opposé à la portion 5.

La portion filetée 5 a un même diamètre et forme un filet identique à celui du pion 54. Elle est apte à recevoir ledit écrou de remplacement 3 vissé sur elle comme décrit plus loin. Elle comprend une portion 5a de section réduite permettant de casser aisément la tige 2 au-delà de la face proximale de l'écrou de remplacement 3 une fois cet écrou mis en place et serré.

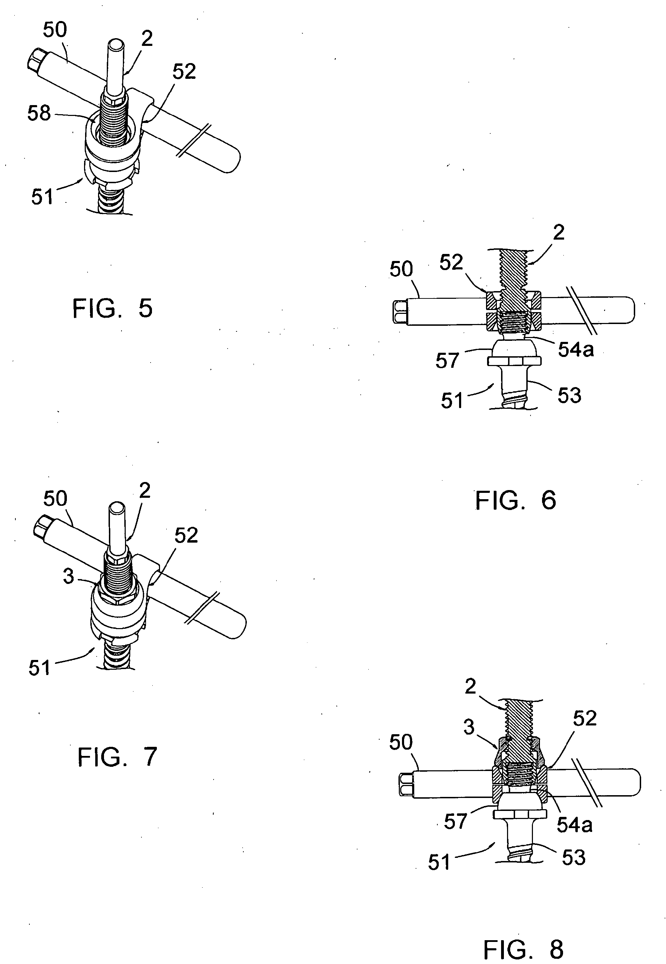

L'extrémité élargie 6 est de forme extérieure circulaire et a un diamètre externe légèrement inférieur au diamètre interne du trou 58. Elle a donc une section transversale telle qu'elle est apte à être engagée au travers de ce trou 58, ainsi que le montrent les figures 5 et 6. L'extrémité 6, grâce à sa forme élargie, délimite une cavité axiale taraudée 10, de diamètre interne correspondant, au jeu fonctionnel près, à celui de la portion subsistante du pion 54, et dont le taraudage est compatible avec le filet que comprend cette portion subsistante 54a. La tige 2 est ainsi apte à être vissée sur cette portion subsistante 54a du pion 54.

La portion à facettes 7 permet une prise en rotation de la tige 2 pour son vissage sur cette portion subsistante 54a.

La portion lisse 8 de plus faible section forme une tige facilitant la descente de l'écrou 3 sur la portion filetée 5.

L'écrou 3 comprend quant à lui une base distale élargie 11 formant une collerette distale définissant une large surface d'appui contre la face proximale de la pièce de connexion 52, et comprend une portion tubulaire distale 12, conique extérieurement, faisant saillie de la base distale élargie 11. Comme cela est visible sur les figures 7 à 10, cette portion tubulaire distale 12 est dimensionnée pour prendre place dans l'espace subsistant entre la pièce de connexion 52, qui forme une cuvette conique proximale au niveau du trou 58, et l'extrémité élargie 6.

En pratique, la reprise du positionnement d'une vis 51 se réalise comme suit.

Le patient est réopéré de manière à accéder à l'écrou d'origine que comporte la vis 51 et cet écrou est desserré puis retiré. La pièce de connexion 52 est écartée de la vis 51 puis cette dernière est retirée et est remise en place de manière adéquate.

Comme le montrent les figures 3 et 4, ce changement de position de la vis 51 par rapport à la pièce de connexion 52, de même que le relâchement de la tension exercée sur la vertèbre par la vis 51, conduit à ce que la portion subsistante 54a du pion 54 ne dépasse pas suffisamment de la pièce 52 pour remettre l'écrou d'origine en place.

La tige filetée de prolongation 2 est alors mise en place par vissage sur cette portion subsistante 54a (cf. figures 5 et 6), par engagement de l'extrémité élargie 6 au travers du trou 58, puis l'écrou de remplacement 3 est mis en place et est serré jusqu'à amener la surface 57 au contact de la pièce de connexion 52 (cf. figures 7 et 8).

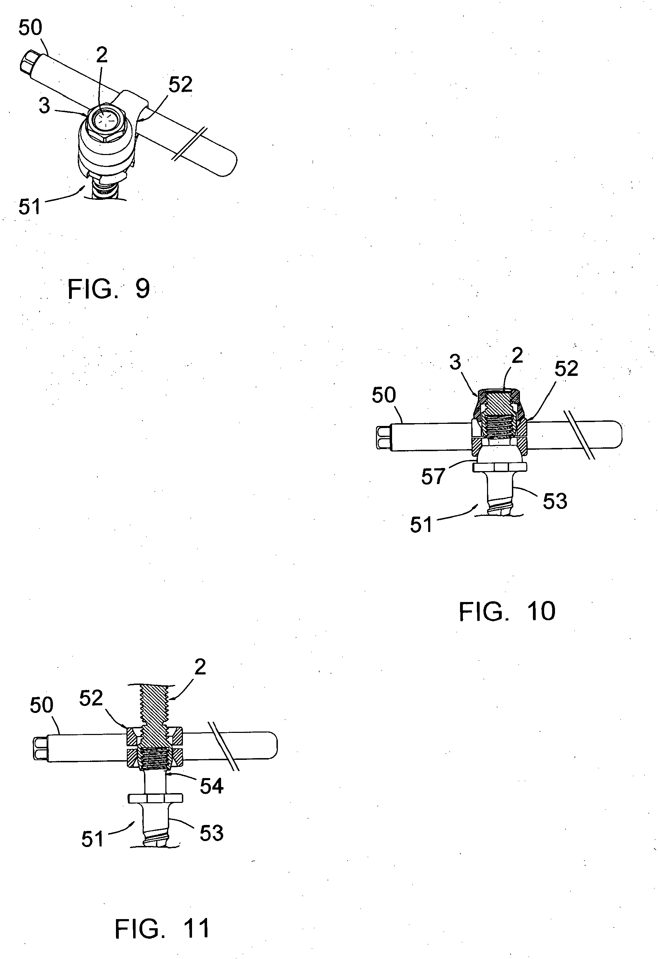

La tige filetée de prolongation 2 est alors cassée au niveau de la portion 5a, laquelle est située à proximité de la face proximale de l'écrou 3 (cf. figures 9 et 10).

La figure 11 montre un matériel très similaire à celui décrit ci-dessus sinon que la vis 51 est "monoaxiale", c'est-à-dire avec un pion proximal fileté 54 formant corps avec l'embase 53.

Comme cela apparaît de ce qui précède, l'invention fournit un ensemble ayant pour avantage déterminant de permettre de réaliser facilement et rapidement une reprise de la position d'une vis pédiculaire.

L'invention a été décrite ci-dessus en référence à une forme de réalisation donnée à titre d'exemple. Il va de soi qu'elle n'est pas limitée à cette forme de réalisation mais qu'elle s'étend à toutes les autres formes de réalisation couvertes par les revendications annexées. En particulier, il a été décrit ci-dessus une vis à pion proximal 54 cassable mais il est clair que l'invention est applicable de la même manière à une vis comprenant un pion proximal court non cassable, c'est-à-dire à un matériel dont la capacité de réduction repose essentiellement sur l'ancillaire associé à ce matériel ; la vis pédiculaire peut être remplacé par un crochet lamaire comprenant un pion proximal fileté.

## Revendications

1. Matériel d'ostéosynthèse vertébrale et ensemble de reprise (1) pour ce matériel d'ostéosynthèse vertébrale, le matériel d'ostéosynthèse vertébrale comprenant au moins un organe d'ancrage osseux (51) incluant un pion proximal fileté (54), au moins une pièce de connexion (52) comprenant un trou (58) pour son engagement sur ce pion proximal fileté (54), et au moins un écrou destiné à être vissé sur le filet que comprend extérieurement le pion (54) pour la réception de cet écrou ;
**caractérisé en ce que** :
- l'ensemble de reprise (1) comprend une tige filetée de prolongation (2) présentant une extrémité élargie (6) qui délimite une cavité axiale taraudée (10), cette cavité axiale taraudée (10) étant dimensionnée pour permettre le vissage de la tige filetée de prolongation (2) sur ledit filet que comprend le pion fileté (54) ;
- le trou (58) que comprend ladite pièce de connexion (52) a un diamètre tel qu'il peut recevoir ladite extrémité élargie (6) de la tige filetée de prolongation (2) au travers de lui ; et
- l'ensemble de reprise (1) comprend un écrou dit "de remontage" apte à être vissé sur cette tige filetée de prolongation (2).

2. Matériel d'ostéosynthèse-vertébrale et ensemble de reprise (1) selon la revendication 1, **caractérisé en ce que** la tige filetée de prolongation (2) a le même diamètre et présente un filet identique à celui du pion proximal fileté (54), et **en ce que** ledit écrou de remontage est l'écrou d'origine que comprend le matériel d'ostéosynthèse.

3. Matériel d'ostéosynthèse vertébrale et ensemble de reprise (1) selon la revendication 1, **caractérisé en ce que** ledit écrou de remontage est un écrou (3) de remplacement de l'écrou d'origine.

4. Matériel d'ostéosynthèse vertébrale et ensemble de reprise (1) selon l'une des revendications 1 à 3, **caractérisé en ce que** ledit organe d'ancrage osseux est une vis pédiculaire "monoaxiale", c'est-à-dire dans laquelle le pion proximal fileté fait corps avec le reste de la vis.

5. Matériel d'ostéosynthèse vertébrale et ensemble de reprise (1) selon l'une des revendications 1 à 3, **caractérisé en ce que** ledit organe d'ancrage osseux est une vis pédiculaire (51) "polyaxiale", c'est-à-dire dans laquelle le pion proximal fileté (54) est articulé par rapport au reste de la vis, le pion proximal fileté comprenant des moyens pour son immobilisation en rotation lors de la mise en place de la tige filetée de prolongation (2).

6. Matériel d'ostéosynthèse vertébrale et ensemble de reprise (1) selon l'une des revendications 1 à 5, **caractérisé en ce que** la tige filetée de prolongation (2) comprend une portion (5a) de section réduite favorisant sa cassure au-delà de la face proximale de l'écroù de remontage après complet serrage de ce dernier.

7. Matériel d'ostéosynthèse vertébrale et ensemble de reprise (1) selon la revendication 3, **caractérisé en ce que** ledit écrou de remplacement (3) a une base distale élargie (11) permettant une prise d'appui contre la face proximale de la pièce de connexion (52).

8. Matériel d'ostéosynthèse vertébrale et ensemble de reprise (1) selon la revendication 7, **caractérisé en ce que** ledit écrou de remplacement (3) comprend une portion distale tubulaire (12), conique extérieurement, faisant saillie de ladite base distale élargie (11) et dimensionnée pour prendre place dans l'espace subsistant entre la pièce de connexion (52) et ladite extrémité élargie (6).

## Patentansprüche

1. Vertebrales Osteosynthese-Material und Anordnung zur Entnahme (1) dieses vertebralen Osteosynthese-Materials, wobei das vertebrale Osteosynthese-Material mindestens ein Knochen-Verankerungsorgan (51), welches einen proximalen, mit Gewinde versehenen Zapfen (54) und mindestens ein Verbindungsteil (52) umfasst, welches für dessen Eingriff auf diesem proximalen, mit Gewinde versehenen Zapfen (54) ein Loch (58) aufweist, und mindestens eine Mutter aufweist, welche dafür vorgesehen ist, auf das Gewinde aufgeschraubt zu werden, welches der Zapfen (54) zur Aufnahme dieser Mutter außen aufweist;
**dadurch gekennzeichnet, dass**:
- die Entnahmeanordnung (1) eine mit Gewinde versehene Verlängerungsstange (2) aufweist, welche ein erweitertes Ende (6) aufweist, das einen axialen, mit Innengewinde versehenen Hohlraum (10) abgrenzt, wobei dieser axiale, mit Innengewinde versehene Hohlraum (10) dimensioniert ist, um das Aufschrauben der mit Gewinde versehenen Verlängerungsstange (2) auf das Gewinde, welches der mit Gewinde versehene Zapfen (54) aufweist, zu ermöglichen;
- das Loch (58), welches das Verbindungsteil (52) aufweist, einen derartigen Durchmesser besitzt, dass es das erweiterte Ende (6) der mit Gewinde versehenen Verlängerungsstange (2) durch sich hindurch aufnehmen kann; und
- die Entnahmeanordnung (1) eine Mutter zum so genannten "Wiederaufbau" aufweist, welche geeignet ist, auf diese mit Gewinde versehene Verlängerungsstange (2) aufgeschraubt zu werden.

2. Vertebrales Osteosynthese-Material und Anordnung zur Entnahme (1) nach Anspruch 1, **dadurch gekennzeichnet, dass** die mit Gewinde versehene Verlängerungsstange (2) denselben Durchmesser besitzt und ein identisches Gewinde aufweist wie jenes des proximalen, mit Gewinde versehenen Zapfens (54), und dass die Mutter zum Wiederaufbau die ursprüngliche Mutter ist, welche das Osteosynthese-Material aufweist.

3. Vertebrales Osteosynthese-Material und Anordnung zur Entnahme (1) nach Anspruch 1, **dadurch gekennzeichnet, dass** die Mutter zum Wiederaufbau eine Mutter (3) zum Austausch der ursprünglichen Mutter ist

4. Vertebrales Osteosynthese-Material und Anordnung zur Entnahme (1) nach einem der Ansprüche 1 bis 3, **dadurch gekennzeichnet, dass** das Knochen-Verankerungsorgan eine "monoaxiale" pedikuläre Schraube ist, das heißt, bei welcher der proximale, mit Gewinde versehene Zapfen einstückig mit dem Rest der Schraube ist.

5. Vertebrales Osteosynthese-Material und Anordnung zur Entnahme (1) nach einem der Ansprüche 1 bis 3, **dadurch gekennzeichnet, dass** das Knochen-Verankerungsorgan eine "polyaxiale" pedikuläre Schraube (51) ist, das heißt, bei welcher der proximale, mit Gewinde versehene Zapfen (54) in Bezug auf den Rest der Schraube gelenkig angeordnet ist, wobei der proximale, mit Gewinde versehene Zapfen Mittel für dessen Drehblockierung beim Anbringen der mit Gewinde versehenen Verlängerungsstange (2) aufweist.

6. Vertebrales Osteosynthese-Material und Anordnung zur Entnahme (1) nach einem der Ansprüche 1 bis 5, **dadurch gekennzeichnet, dass** die mit Gewinde versehene Verlängerungsstange (2) einen Abschnitt (5a) mit geringerem Querschnitt aufweist, welcher ihren Bruch oberhalb der proximalen Fläche der Mutter zum Wiederaufbau begünstigt, nachdem diese Letztere vollständig angezogen worden ist.

7. Vertebrales Osteosynthese-Material und Anordnung zur Entnahme (1) nach Anspruch 3, **dadurch gekennzeichnet, dass** die Austauschmutter (3) eine erweiterte, distale Basis (11) besitzt, welche ein Abstützen an der proximalen Fläche des Verbindungsteils (52) ermöglicht.

8. Vertebrales Osteosynthese-Material und Anordnung zur Entnahme (1) nach Anspruch 7, **dadurch gekennzeichnet, dass** die Austauschmutter (3) einen distalen, rohrförmigen, außen konischen Abschnitt (12) aufweist, welcher von der erweiterten, distalen Basis (11) vorspringt und dimensioniert ist, um sich in den zwischen dem Verbindungsteil (52) und dem erweiterten Ende (6) bestehenden Spalt einzufügen.

## Claims

1. Vertebral osteosynthesis equipment and revision assembly (1) for this vertebral osteosynthesis equipment, the vertebral osteosynthesis equipment comprising at least one bone anchoring member (51) including a threaded proximal pin (54), at least one connecting part (52) comprising a hole (58) for its engagement on that threaded proximal pin (54), and at least one nut designed to be screwed on the thread outwardly comprised by the pin (54) for receiving that nut;
**characterized in that**:
- the revision assembly (1) comprises a threaded extension rod (2) having a widened end (6) that defines a threaded axial cavity (10), said threaded axial cavity (10) being dimensioned to allow the threaded extension rod (2) to be screwed onto said thread provided on the threaded pin (54);
- the hole (58) provided in said connecting part (52) has a diameter such that it is capable to receive said widened end (6) of the threaded extension rod (2) therethrough; and
- the revision assembly (1) comprises a so-called "reassembly" nut suitable for being screwed onto said threaded extension rod (2).

2. Vertebral osteosynthesis equipment and revision assembly (1) according to claim 1, **characterized in that** the threaded extension rod (2) has the same diameter and has a thread identical to that of the threaded proximal pin (54) and **in that** the reassembly nut is the original nut included in the osteosynthesis equipment.

3. Vertebral osteosynthesis equipment and revision assembly (1) according to claim 1, **characterized in that** said reassembly nut is a nut (3) for the replacement of the original nut.

4. Vertebral osteosynthesis equipment and revision assembly (1) according to one of claims 1-3, **characterized in that** said anchoring member is a "mono-axial" pedicle screw, i.e., in which the threaded proximal pin is integral with the rest of screw.

5. Vertebral osteosynthesis equipment and revision assembly (1) according to one of claims 1-3, **characterized in that** said anchoring member is a "polyaxial" pedicle screw, i.e., in which the threaded proximal pin (54) is articulated relative to the rest of the screw, the threaded proximal pin comprising means for immobilizing its rotation during placement of the threaded extension rod (2).

6. Vertebral osteosynthesis equipment and revision assembly (1) according to one of claims 1-5, **characterized in that** the threaded extension rod (2) comprises a portion (5a) with a reduced section favoring its breakage beyond the proximal face of the reassembly nut, after the latter is completely tightened.

7. Vertebral osteosynthesis equipment and revision assembly (1) according to claim 3, **characterized in that** said replacement nut (3) comprises a widened distal base (11) allowing bearing against the proximal face of the connecting part (52).

8. Vertebral osteosynthesis equipment and revision assembly (1) according to claim 7, **characterized in that** said replacement nut (3) comprises a tubular, outwardly conical distal portion (12), protruding from said widened distal base (11) and dimensioned to be placed in the space existing between the connecting part (52) and said widened end (6).
